# EUROPEAN PATENT SPECIFICATION

(11) **EP 2 910 944 B1**
(45) Date of publication and mention of the grant of the patent: **26.09.2018**
(21) Application number: 13849029.7
(22) Date of filing: 22.10.2013
(51) Int. Cl.: G01N 33/49, B01L 3/00, G01N 1/40

(54) **FILTER DEVICE**
FILTERVORRICHTUNG
DISPOSITIF À FILTRE

(30) Priority: 22.10.2012 JP 2012232592; 21.01.2013 JP 2013008139
(43) Date of publication of application: 26.08.2015
(73) Proprietor: Panasonic Intellectual Property Management Co., Ltd., Osaka-shi, Osaka 540-6207 (JP)
(72) Inventor: NOGAMI, Takahiro, Osaka-shi Osaka 540-6207 (JP); YAMAMOTO, Takeki, Osaka-shi Osaka 540-6207 (JP); SESEI, Takashi, Osaka-shi Osaka 540-6207 (JP)
(74) Representative: Grünecker Patent- und Rechtsanwälte PartG mbB
(86) International application number: PCT/JP2013/006232
(87) International publication number: WO 2014/064921

(56) References cited:
- WO-A1-2012/085006
- DE-A1- 4 132 480
- JP-A- 2003 135 435
- JP-A- 2010 518 393
- US-A- 3 969 250
- US-A1- 2010 224 078
- US-A1- 2011 008 908

## Description

### TECHNICAL FIELD

The present invention relates to a blood filter device for filtering a liquid.

### BACKGROUND ART

WO 2012/085006 A1 which is regarded as the closest prior art document relates to filtration devices and methods for filtering liquid samples and in particular, to filtration devices in which a plunger including a filter material is inserted into a liquid receptacle. Said filtration device comprises a liquid receptacle including an open end and a closed end, a plunger body movable at least partially within the liquid receptacle along an axis. Said axis extends between the open and closed ends. Said plunger includes a filter chamber in fluid communication with the liquid receptacle via a fluid filtering path. Moreover, the filtration device comprises a filter disposed in the filtering path and a slidable seal. The plunger body includes a cap attached to or forming part of the body.

US 2011/008908 A1 refers to an apparatus and a method for separating and analyzing blood.

DE 41 32 480 A1 discloses an apparatus for blood collection with a cylindrical sample tube in which an axially directed cone is formed. Moreover, this document discloses a cap and check valves.

US 3 969 250 A refers to a device for filtering, isolating, containing and storing fluid specimens which are to be analyzed with an automatic chemical analyzer.

FIG. 14 is a cross-sectional view of conventional filter device 1 disclosed in PTL 1. Filter device 1 selectively removes white blood cells and degenerated blood components from blood cells suspended in a liquid, such as a blood or a body fluid. Filter device 1 includes container 4 having inlet 2 and outlet 3 for blood cells suspended in the liquid, and filter part 5 provided inside container 4. Filter part 5 includes a degenerated-blood-component removing filter and a white-blood-cell removing filter made of non-woven fabric. For example, a tube is connected to inlet 2. White blood cells and degenerated blood components are selectively removed such that the blood cells suspended in the liquid are injected into container 4 through inlet 2, and is pressurized from upstream of the blood cells suspended in the liquid, and white blood cells and degenerated blood components are captured as captured substances with filter part 5.

### CITATION LIST

### PATENT LITERATURE

PTL 1: Japanese Patent Laid-Open Publication No.60-203267

### SUMMARY

It is an object of the present invention to provide a blood filter device, wherein the stabling of the capturing performance of the filter part can be improved.

The above and other objects of the invention are achieved by the blood filter device according to claim 1. Preferred embodiments are claimed in the dependent claims.
The blood filter device according to claim 1 is configured to filter a sample containing a liquid component and a plurality of solid components mixed with the liquid component. The blood filter device comprises: a container configured to store the sample therein; a tube configured to be inserted into the container; and a filter part provided inside the tube. When the tube is inserted into the container, the filter part is configured to allow the stored sample to pass through the filter part so as to capture the plurality of solid components and to allow the liquid component to pass through the filter part.

Moreover, the blood filter device comprises a capturing-retaining mechanism configured to prevent the plurality of captured solid components from being removed from the filter part when the sample passes through the filter part; and a cap provided at an opening end of the tube opposite to an opening end to which the filter part is joined and closing the opening end of the tube. The cap is configured to be removable from the opening end of the tube. The cap is configured to hermetically seal the container to increase an inner pressure of the container when the tube is inserted into the container. The container has an opening having the tube inserted therein.

The capturing-retaining mechanism is structured such that: the capturing-retaining mechanism includes a first projection provided on an outer circumference of the tube and surrounding the outer circumference of the tube, the first projection having an outermost diameter not smaller than an inner diameter of the container, and a second projection provided on the tube, the second projection being configured to contact an end portion of the container facing the opening so as to prevent the tube from being inserted into the container by a depth not smaller than a predetermined depth, or the capturing-retaining mechanism includes: a projection provided on an outer circumference of the tube; and a stopper provided on an inner wall of the container, the stopper being configured to contact the projection when the tube is inserted into the container.

According to claim 2, the capturing-retaining mechanism is configured such that a portion of the sample does not pass through the filter part and remains in the container when the tube is inserted into the container.

According to claim 3, a compressed volume of the container when the tube is inserted into the container is not larger than an amount of the sample that passes through the filter part.

The blood filter device has high filter performance.

### BRIEF DESCRIPTION OF DRAWINGS

FIG. 1 is a cross-sectional view of a filter device according to Exemplary Embodiment 1 of the present invention.
FIG. 2A is an enlarged cross-sectional view of the filter device according to Embodiment 1.
FIG. 2B is an enlarged cross-sectional view of the filter device according to Embodiment 1.
FIG. 3 is a cross-sectional view of another container of the filter device according to Embodiment 1.
FIG. 4A is a cross-sectional view of the filter device according to Embodiment 1 for illustrating a filtering method used with the filter device.
FIG. 4B is a cross-sectional view of the filter device according to Embodiment 1 for illustrating the filtering method used with the filter device.
FIG. 4C is a cross-sectional view of the filter device according to Embodiment 1 for illustrating the filtering method used with the filter device.
FIG. 4D is a cross-sectional view of the filter device according to Embodiment 1 for illustrating the filtering method used with the filter device.
FIG. 5 is a cross-sectional view of another filter device according to Embodiment 1.
FIG. 6 is a cross-sectional view of a filter device according to Exemplary Embodiment 2 of the present invention.
FIG. 7 is a cross-sectional view of the filter device according to Embodiment 2.
FIG. 8A is a cross-sectional view of the filter device according to Embodiment 3 for illustrating a filtering method used with the filter device.
FIG. 8B is a cross-sectional view of the filter device according to Embodiment 3 for illustrating the filtering method used with the filter device.
FIG. 8C is a cross-sectional view of the filter device according to Embodiment 3 for illustrating the filtering method used with the filter device.
FIG. 9 is a cross-sectional view of the filter device according to Embodiment 2.
FIG. 10 is a cross-sectional view of a filter device according to Example 1 which does not form part of the invention but is useful for understanding the invention.
FIG. 11A is a cross-sectional view of another filter device according to Example 1 which does not form part of the invention but is useful for understanding the invention.
FIG. 11B is a cross-sectional view of still another filter device according to Example 1 which does not form part of the invention but is useful for understanding the invention.
FIG. 12 is a cross-sectional view of a filter device according to Example 2 which does not form part of the invention but is useful for understanding the invention.
FIG. 13 is a cross-sectional view of another filter device according to Example 2 which does not form part of the invention but is useful for understanding the invention.
FIG. 14 is a cross-sectional view of a conventional filter device.

### DETAILED DESCRIPTION OF PREFERRED EMBODIMENTS EXEMPLARY EMBODIMENT 1

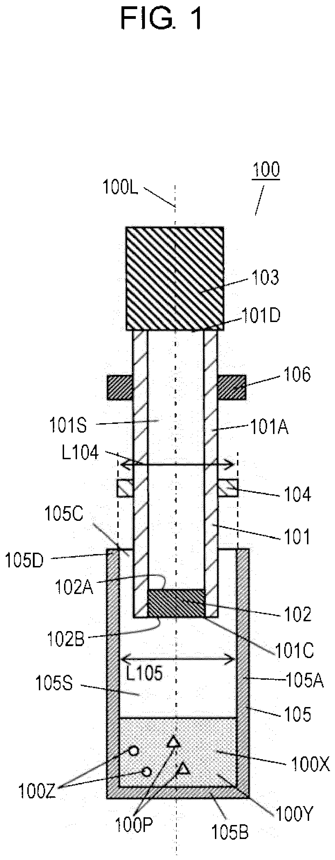

FIG. 1 is a cross-sectional view of filter device 100 according to Exemplary Embodiment 1. Filter device 100 which is a blood filter device includes container 105 having a tubular shape having side wall 105A extending along reference axis 100L, and bottom 105B positioned on reference axis 100L, and has opening 105C. Container 105 has inner space 105S configured to store sample 100X therein. Sample 100X contains liquid component 100Y and plural solid components 100P and 100Z which are mixed and suspended in liquid component 100Y. Filter device 100 is configured to filter sample 100X so as to remove solid components 100Z as filtered-out substances from sample 100X and to allow liquid component 100Y and solid components 100P as a filtrate to pass through the filter device. Solid components 100Z are filtered-out substances and are filtered out by filter device 100 while solid components 100P are non-filtered-out substances which are not filtered out by filter device 100. Filter device 100 includes container 105, tube 101 configured to be inserted into inner space 105S of container 105, and filter part 102 provided inside tube 101. Tube 101 extends along reference axis 100L, and has opening ends 101C and 101D opposite to each other on reference axis 100L and inner space 101S communicating with the outside of tube 101 through opening ends 101C and 101D. Filter part 102 is located near opening end 101C or 101D of tube 101, and closes tube 101. Cap 103 closes opening end 101D of tube 101. When tube 101 is inserted into inner space 105S of container 105 and reaches sample 100X, filter part 102 captures solid components 100Z so as not to allow solid components 100Z to pass through filter part 102 while filter part 102 does not capture solid components 100P so as to allow solid components 100P to pass through filter part 102. Filter device 100 includes a capturing-retaining mechanism configured to prevent solid components 100Z captured by filter part 102 from being released and removed from filter part 102. As shown in FIG. 1, for example, the capturing-retaining mechanism is configured such that, when tube 101 is inserted into container 105, an average mobility of solid components 100Z in filter part 102 is constant. More specifically, the capturing-retaining mechanism allows sample 100X to pass through filter part 102 at a constant pressure when tube 101 is inserted into container 105.

Solid components 100Z are captured by filter part 102 by chemical bonding or physical capturing. A kind of the chemical bonding is not particularly limited, and the chemical bonding may be, e.g. covalent bonding, ionic bonding, hydrogen bonding, bonding by Van der Waals force. When solid components 100Z are made of a biomaterial, such bonding may include an adhesion by membrane protein contained in a biomaterial inherently. On the other hand, the physical capturing refers to capturing based on a structural factor, such as a size of pores in the material, of a material for forming filter part 102.

The average mobility of solid components 100Z in filter part 102 refers to an average of moving speed of solid components 100Z in response to an external force which acts on solid components 100Z in filter part 102. Filter device 100 according to Embodiment 1 is operable such that the average mobility is constant.

A pressure which is applied to sample 100X when sample 100X passes through filter part 102 is constant within a range where filter part 102 can maintain a capturing force for capturing solid components 100Z when the filtered-out substances, that is, solid components 100Z are filtered out. The pressure does not rapidly change. For example, in the case that solid components 100Z are white blood cells, the pressure ranges from several kPa to several tens kPa.

The shape of a cross section of tube 101 along a line perpendicular to reference axis 100L may be a circular shape, an elliptical shape, a rectangular shape, a trapezoidal shape, a polygonal shape, or an arbitrary shape surrounded by a closed curve.

Tube 101 is made of, for example, glass, such as quartz glass, borosilicate glass, soda-lime glass, potash glass, crystal glass, uranium glass, or acrylic glass, or made of a resin, such as polypropylene, polyurethane, polyvinyl chloride, polyvinyl acetate, polytetrafluoroethylene, acrylonitrile butadiene styrene, polyacetal, polybutylene terephthalate, polyolefin, polystyrene, polydimethylsiloxane, polyamide, polycarbonate, polyethylene terephthalate, polyphenylene sulfide, polyether ether ketone, or polymethyl methacrylate.

An inner wall of tube 101 preferably has hydrophilicity. The inner wall of tube 101 may preferably have high hydrophilicity. In order to provide the inner wall of tube 101 with hydrophilicity, the following treatment may be used. Treatment may be, for example, plasma treatment with an oxygen gas or the like, the formation of composites made of hydrophilic nanoparticles or a surfactant, the formation of ultrafine unevenness, the formation of a silica-based film containing water glass or the like as a main component, the formation of a resin-based film using various hydrophilicity resins (water-soluble photosensitive resins), a wet process using a drastic medicine such as chromic anhydride or a sulfuric acid, optical surface treatment with ultra violet rays (UV ozone method), treatment with a siloxane-based static electricity prevention agent, or electrolytic polymer coating. As a method for maintaining hydrophilicity, tube 101 is stored at lower temperature, in freezing state, or in water.

Filter part 102 is provided on the inner wall of tube 101. Filter part 102 is provided at opening end 101C or near opening end 101C of tube 101. Filter part 102 provided at such a position allows tube 101 to collect liquid component 100Y which is filtered in inner space 101S of tube 101 so that tube 101 can be effectively used as a collecting container.

Filter part 102 is joined to tube 101 such that a leakage can be prevented.

Filter part 102 may be made of nano-fiber, non-woven fabric, or a porous body made of polymer, an organic compound, an inorganic compound, or an inorganic oxide.

Filter part 102 may be made of a silicon oxide which mainly contains silicon oxide. Filter part 102 is preferably made of amorphous silicon dioxide. Filter part 102 may be made of glass, such as borosilicate glass or soda-lime glass, or made of a resin, such as polypropylene, polyurethane, polyvinyl chloride, polyvinyl acetatepolyacetal, or polystyrene, besides silicon dioxide. The material for forming filter part 102 is not limited to above materials.

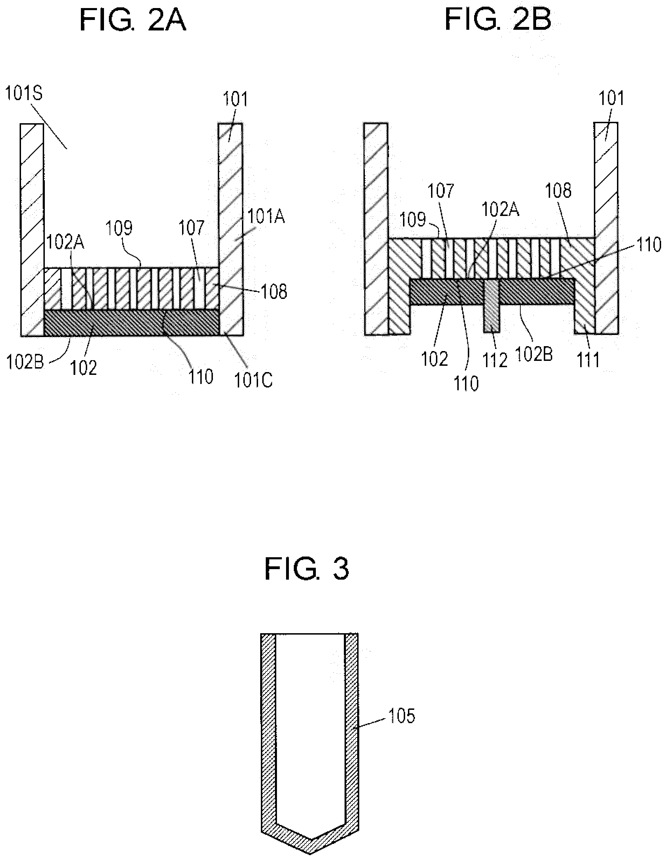

FIG. 2A is an enlarged cross-sectional view of filter device 100. Filter part 102 has surface 102A and surface 102B opposite to surface 102A. Surface 102A faces inner space 101S of tube 101. Filter device 100 may include thin plate 108 provided on surface 102A of filter part 102. Thin plate 108 has surface 110 and surface 109 opposite to surface 110. Surface 110 is positioned on surface 102A of filter part 102. At least one aperture 107 which passes through thin plate 108 from surface 109 to surface 110 is formed in thin plate 108. Filter part 102 covers surface 110 of thin plate 108 and openings of apertures 107.

Thin plate 108 provided on surface 102A of filter part 102 can increase strength of filter part 102. Thin plate 108 may be provided on surface 102B of filter part 102, thereby providing the same effects. Plural apertures 107 may be formed in thin plate 108. The number of apertures 107 is determined corresponding to the application where filtering is used.

Filter part 102 and thin plate 108 be preferably bonded to each other. Filter part 102 and thin plate 108 bonded to each other can prevent filter part 102 from moving during filtering. Filter part 102 and thin plate 108 may preferably be directly bonded to each other. According to this embodiment, "filter part 102 and thin plate 108 are directly bonded to each other" refers to a state where filter part 102 is directly formed on thin plate 108 and atoms or molecules which constitute thin plate 108 and atoms or molecules which constitute filter part 102 are directly bonded to each other. The clause, "filter part 102 and thin plate 108 are directly bonded to each other" often refers to a state where covalent bonding is made between atoms. For example, when thin plate 108 is made of silicon, filter part 102 is made of fibrous substances made of silicon. Filter part 102 made of the fibrous substances made of silicon allows silicon atoms of thin plate 108 to be bonded to silicon atoms in the fibrous substances by covalent bonding along with oxygen molecules in an atmosphere where the fibrous substances are formed, and thereby, filter part 102 and thin plate 108 can be directly bonded to each other.

Filter part 102 and thin plate 108 may be indirectly bonded to each other with an adhesive agent.

FIG. 2B is an enlarged cross-sectional view of filter device 100. Filter device 100 may further include wall part 111 having an annular shape surrounding the entire periphery of filter part 102 along tube 101. Wall part 111 formed unitarily with the periphery of filter part 102 allows filter part 102 to be easily held and bonded to tube 101 when filter part 102 is installed to tube 101. Wall part 111 has a shape which conforms with a shape of filter part 102 so as to continuously surround the periphery of filter part 102. The shape of wall part 111 may be a circular annular shape or a polygonal annular shape, such as a triangular annular shape or a rectangular annular shape.

Reinforcing part 112 may be formed on portions of surface 110 of thin plate 108 except for wall part 111 and apertures 107 such that reinforcing part 112 is connected with thin plate 108. Reinforcing part 112 has a plate shape extending in a direction perpendicular to the surface of thin plate 108, for example, and reinforcing part 112 is also connected to a side surface of wall part 111. This configuration allows a pressure applied to thin plate 108 to be further dispersed, hence preventing breakage of thin plate 108. From this point of view, reinforcing part 112 is preferably provided on surface 110 of thin plate 108. Plural reinforcing parts 112 may be provided, thereby improving pressure resistance of thin plate 108 against a force applied to thin plate 108 in the direction perpendicular to thin plate 108.

When filter part 102 is formed using with fibrous substances made of a silicon oxide which mainly contains silicon oxide, a Silicon On Insulator (SOI) substrate in which a silicon layer is made of silicon (100) as a substrate is preferably used for manufacturing filter part 102. The SOI substrate has a three-layered structure including a silicon layer, a silicon dioxide layer provided on the silicon layer, and another silicon layer provide on the silicon dioxide layer so as to face the silicon layer across the silicon dioxide layer. By applying microfabrication to the SOI substrate using photo lithography and an etching technique, it is possible to collectively manufacture a large number of filter parts 102 each having thin plate 108.

In the SOI substrate, the silicon dioxide layer can function as an etching-stop layer which stops etching during performing etching processing. Further, the silicon dioxide layer has high hydrophilicity, and hence, can easily suppress the generation of bubbles when sample 100X passes through filter part 102, and can easily remove the generated bubbles. Accordingly, the measurement with high accuracy can be realized. The thickness of silicon dioxide layer may preferably range from 0.5 to 10 µm from a viewpoint of a thickness which the silicon dioxide layer is required to have as an etching-stop layer and productivity.

In general, a silicon dioxide layer which functions as the etching-stop layer is made of a silicon dioxide layer which is formed by thermal oxidation. The silicon dioxide layer may be made of a silicon dioxide layer which is formed by other methods, such as a chemical vapor deposition (CVD) method, a sputtering method, or a chemical solution deposition (CSD) method or a doped oxide layer such as a so-called phosphorus silicon glass (PSG) layer doped with phosphorus or a boron phosphorus silicon glass (BPSG) layer which is doped with phosphorus and boron. Further, the etching-stop layer is not limited to the above-mentioned layer which mainly contains silicon dioxide. A layer made of inorganic oxide or inorganic nitride, such as silicon nitride, silicon oxynitride or aluminum oxide, having an etching rate greatly different from an etching rate of silicon may function as the etching-stop layer.

According to Embodiment 1, the substrate for manufacturing filter part 102 is an SOI substrate which contains silicon (100). However, the substrate for manufacturing filter part 102 may be a silicon (110) substrate, a silicon (111) substrate, or a silicon substrate having the different plane orientation. Particularly, an amorphous silicon substrate used as the substrate can suppress a phenomenon that thin plate 108 which receives a largest force at the time of filtering is liable to be broken along the plane orientation. Further, as the substrate for manufacturing filter part 102, other substrates, such as a glass substrate or a substrate made of a film resin, may be used besides the silicon substrate. From a viewpoint of workability and general-purpose use property, a substrate which contains silicon (100) can be used as the substrate for manufacturing filter part 102.

By increasing a thickness of thin plate 108, thin plate 108 which is liable to receive a force during filtering is hardly broken. However, there arises a problem with respect to an aspect ratio in a Bosch process or the like in forming minute apertures 107. Accordingly, the thickness of thin plate 108 may preferably range approximately from 5 to 150 µm.

A desired number can be selected as the number of apertures 107. By changing the number of mask holes formed in a resist mask which is formed before apertures 107 are formed, it is possible to set the number of apertures 107 to a desired number by using the substantially same process.

In the case that the number of apertures 107 is large, a filtered sample or sample 100X can easily pass through filter part 102. By increasing the number of apertures 107, the same amount of solution can be filtered for a short time without increasing an area of filter part 102 and a filtering time so that an operation efficiency of filter device 100 is enhanced. Accordingly, the number of apertures 107 is preferably large. Plural apertures 107 formed in surface 109 of thin plate 108 may preferably be arranged in a honeycomb pattern. This arrangement nay increase the number of apertures 107 formed in thin plate 108 per unit area without reducing strength of thin plate 108.

The diameter of aperture 107 can be adjusted to a value suitable for suppressing the generation of resistance in a flow passage when sample 100X passes through aperture 107. For example, in the case that blood or a living-organism-derived solution containing red blood cells is used as sample 100X, and red blood cells are to be extracted in filtrate 100Q as solid components 100P (non-filtered-out substances), the diameter of aperture 107 may preferably be not smaller than 3 µm.

In the case that filter part 102 is formed using the fibrous substances made of silicon oxide which mainly contains silicon oxide, filter part 102 may preferably be manufactured by a vaporized substrate deposition (VSD) method or a vapor liquid solid (VLS) method. For example, in the case that the fibrous substances made of silicon dioxide are manufactured by a VSD method, filter part 102 is manufactured using an oxidative gas, such as oxygen or ozone, and a material which mainly contains silicon.

By performing heat treatment in an atmosphere of high temperature ranging from, e.g. 900°C to 1500°C and low oxygen concentration, silicon monoxide is evaporated from the material mainly containing silicon, and thereafter, the evaporated silicon monoxide adheres onto a surface of the material again and coagulates so that silicon dioxide can grow. At this moment, while silicon monoxide spreads on the entire surface of the silicon layer, silicon monoxide locally adheres again to places where a catalyst layer is formed, and is bonded to oxygen so that fibrous substances mainly containing silicon dioxide grows from these places.

Here, "atmosphere of low oxygen concentration" refers to an atmosphere at the time of performing heat treatment where an oxygen partial pressure is low. Even in such a reduced-pressure atmosphere where the pressure is lower than the atmospheric pressure, oxygen may be replaced with another gas. This gas may be, e.g. nitride, argon, carbon or monoxide. Unlike oxygen and ozone, these gasses have low oxidizability. When a partial pressure of oxygen is extremely low, silicon monoxide cannot be generated. Accordingly, a partial pressure of oxygen preferably ranges from 10⁻² Pa to several thousands Pa.

The thickness of the fibrous substances of filter part 102 may preferably range approximately from 0.01 to 2.0 µm. The fibrous substances are directly bonded to thin plate 108, and are formed densely such that the fibrous substances are entangled with each other. Fibrous substances which are branched in various directions may be mixed in the fibrous substances.

Since fibrous substances are entangled with each other and some fibrous substances are branched, filter part 102 is strongly or firmly formed on surface 109 of thin plate 108. Further, the fibrous substances are entangled with each other in bent shapes so that gaps are formed between the fibrous substances such that the gaps are easily filled with solid components 100Z from various directions including areas above the openings of apertures 107 formed in surface 109. The thickness of fibrous substances is not limited to a constant, and the fibrous substances may include fibrous substances having various thicknesses.

The thickness of the fibrous substances may preferably be gradually increased in the direction toward thin plate 108 from distal ends of the fibrous substance.

The shortest distance of the gap between the fibrous substances is smaller than a diameter of solid component 100Z to be captured.

Solid components 100Z out of solid components 100Z contained in sample 100X having a maximum diameter larger than a size of pores formed between the fibrous substances are captured by the fibrous substances while solid components 100P having a maximum diameter smaller than the size of the pores formed between the fibrous substances pass through the fibrous substances as non-filtered-out substances. This configuration can separate the non-filtered-out substances (solid components 100P) from the filtered-out substances (solid components 100Z) in sample 100X. Even in the case that the maximum diameter of the non-filtered-out substances (solid components 100P) is larger than the size of the pores formed between the fibrous substances, if the non-filtered-out substances (solid components 100P) can easily deform, since the non-filtered-out substances (solid components 100P) deform in the course of passing through the pores formed between the fibrous substances, the non-filtered-out substances (solid components 100P) can be extracted into the filtrate.

In the case that thin plate 108 having apertures 107 is provided on an upper surface of filter part 102 and the non-filtered-out substances (solid components 100P) are also deformable, solid components 100P can pass through apertures 107 even when the maximum diameter of the non-filtered-out substances (solid components 100P) is larger than the diameter of apertures 107. For example, when blood is used as sample 100X, white blood cells are extracted as filtered-out substances (solid components 100Z) and red blood cells are extracted as non-filtered-out substances (solid components 100P), the size of pores formed between the fibrous substances may preferably a value ranging from 1 µm to 6µm. The size of pores ranging from 1µm to 6µm allows filter part 102 to pass only red blood cells which are the non-filtered-out substances through filter part 102 while filter part 102 can prevent white blood cells which are the filtered-out substances from passing through filter part 102 by capturing the white blood cells. In order to completely allow red blood cells which are the non-filtered-out substances to pass through filter part 102, the diameter of apertures 107 is preferably not smaller than 3µm.

The thickness of filter part 102 preferably ranges from 1µm to 1000µm. However, the thickness of filter part 102 may be not smaller than 1000 µm according to the amount of the filtered-out substances. In the case that the fibrous substances are used for forming filter part 102, the formation of layers is not limited to a single layer, and fibrous substances made of different materials or having different shapes may be laminated to each other.

Surface treatment may be applied to filter part 102. That is, the treatment such as the formation of a film by a coupling reaction using an organic silane-based compound, an organophosphonate compound, or a resin-based compound or electrolytic polymer coating by a mutual lamination method may be applied to filter part 102.

Container 105 for storing sample 100X has a bottom, and has a tubular shape. As long as tube 101 can be inserted into container 105, the size of container 105 is not particularly limited.

Container 105 is made of, for example, glass, such as quartz glass, borosilicate glass, soda-lime glass, potash glass, crystal glass, uranium glass or acrylic glass, or a resin, such as polypropylene, polyurethane, polyvinyl chloride, polyvinyl acetate, polytetrafluoroethylene, acrylonitrile butadiene styrene, polyacetal, polybutylene terephthalate, polyolefin, polystyrene, polydimethyl siloxane, polyamide, polycarbonate, polyethylene terephthalate, polyphenylene sulfide, polyetheretherketone or polymethyl methacrylate.

As a volume of container 105 is larger, a pressure in container 105 can be more easily adjusted during filtering. In this case, however, as the increase of the volume of container 105, the amount of sample 100X which remains in container 105 is increased. FIG. 3 is a cross-sectional view of another container 105 of filter device 100 according to Embodiment 1. A bottom surface of container 105 has a conical shape such that the center of the bottom surface projects toward the outside of the container. The bottom surface of container 105 having such a shape can reduce an amount of remaining sample 100X. The bottom surface of container 105 may have a hemispherical shape.

In the case that the bottom surface of container 105 has a conical shape, when a tapered angle (an angle formed by a side surface of a conical shape and the bottom surface) is extremely large, that is, when a vertex angle θ of the conical shape is small, the amount of remaining sample 100X becomes large. Accordingly, such a shape with a small vertex angle is not preferable.

A material of container 105 is not limited. However, when hydrophobicity is imparted to a surface of the inner wall of container 105, a large angle of wettability can be maintained (a meniscus can be maintained in a convex shape) and hence, sample 100X can continue contacting filter part 102 even when an amount of sample 100X is small. Accordingly, it is preferable to use a material which exhibits hydrophobicity. A contact angle formed with the surface of the inner wall of container 105 may preferably be not smaller than 60 degrees.

Sample 100X is a liquid containing solid components 100P and 100Z. Solid components 100Z which are target components are trapped by filter part 102. Sample 100X contains blood and a material derived from, e.g. living organisms.

The capturing-retaining mechanism configured to allow sample 100X to pass through filter part 102 at a constant pressure when tube 101 is inserted into container 105 includes, e.g. projection 104 formed on an outer circumference of tube 101, as shown in FIG. 1. Projection 104 surrounds a peripheral edge of the outer circumference of tube 101.

The shape of an outer circumference of projection 104 is substantially identical to the shape of an inner circumference of container 105. For example, as shown in FIG. 1, the outer circumference of projection 104 is a circular shape.

Projection 104 is formed on the peripheral edge of the outer circumference of tube 101. Alternatively, a recess indented toward the inside of tube 101 may be formed in the peripheral edge of the outer circumference of tube 101. In this case, projection 104 is formed on the recess.

Outermost diameter L104 of projection 104 is not smaller than inner diameter L105 of container 105. That is, in order to prevent sample 100X and air from leaking from the outer periphery of projection 104 when tube 101 is inserted into container 105, projection 104 has a size allowing a side surface of projection 104 to enter in container 105. Projection 104 resiliently contacts an inner wall of container 105 and hermetically closes a space in container 105 together with an outer wall surface of tube 101.

Projection 104 is made of a deformable material, that is, a material having resiliency. For example, projection 104 is made of an O-ring. Projection 104 is made of, e.g. a resilient rubber, such as silicone rubber, fluororubber, urethane rubber, natural rubber, chloroprene rubber, nitrile rubber, ethylene-propylene rubber, butyl rubber, chlorosulfonated polyethylene rubber, acrylic rubber, isoprene rubber, epichlorohydrin rubber, or butadiene rubber.

When tube 101 is inserted into container 105 from the opening, projection 104 is inserted into container 105 so that a space defined in container 105 can be hermetically sealed.

Projection 106 is provided on the outer circumference of tube 101 at a position above projection 104. When tube 101 is inserted into container 105, projection 106 is stopped at an end portion of the opening of container 105. This prevents tube 101 from being inserted further into container 105.

The distance between projection 104 and projection 106 is fixed, and hence, variations in pressure in a hermetically sealed space of respective filter devices 100 can be reduced before filtering.

According to Embodiment 1, projection 106 is stopped at a flat surface at the end portion of container 105. An undulation which fits to projection 106 may be formed in the end portion of container 105. Alternatively, projection 106 and the end portion of the opening of container 105 may have a threaded structure.

The position of projection 106 is determined such that sample 100X filled in container 105 does not go beyond projection 104 when tube 101 is inserted into container 105.

When tube 101 is inserted into container 105 and is stopped at the end portion of container 105, a lower surface of filter part 102 may not preferably reach the bottom surface of container 105. When the lower surface of filter part 102 contacts the bottom surface of container 105, filtering efficiency is deteriorated.

Cap 103 is provided at opening end 101D of tube 101 opposite to opening end 101C to which filter part 102 is joined. Cap 103 is easily removable from opening end 101D of tube 101. Cap 103 closes opening end 101D of tube 101.

Cap 103 can increase an inner pressure of container 105 without causing filter part 102 to contact sample 100X when tube 101 is inserted into container 105. This configuration can reduce a length of container 105. After the inner pressure of container 105 rises to a predetermined pressure, opening end 101D of tube 101 is opened by removing cap 103, and sample 100X can be filtered at a predetermined pressure, hence stabling the capturing performance of filter part 102.

For the same reason, filter device 100 may include a metal seal, a rubber plug, a leak valve, or a leak hole which can close and open opening end 101D of tube 101 instead of cap 103.

In the case that the outer diameter of cap 103 is larger than the outer diameter of tube 101, cap 103 is stopped at the end portion of opening of container 105. In this case, cap 103 functions as projection 106 even when filter device 100 does not include projection 106.

An operation of filter device 100 will be described below.

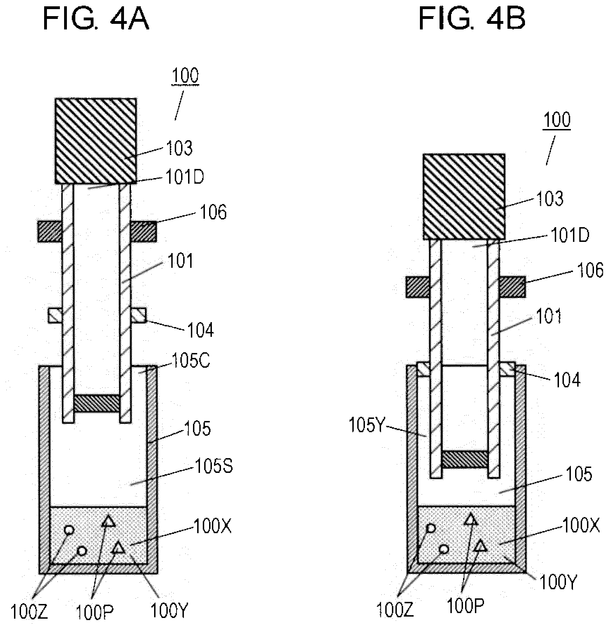

FIGs. 4A to 4D are cross-sectional views of filter device 100 according to Embodiment 1 for illustrating a filtering method used with filter device 100.

As shown in FIG. 4A, sample 100X is put in container 105. Then, tube 101 is inserted into container 105 through upper opening 105C of container 105 provided at the upper part of container 105.

As shown in FIG. 4B, projection 104 formed on tube 101 is inserted into container 105 so that hermetically sealed space 105Y is formed by sample 100X in container 105, projection 104, and the outer surface of tube 101.

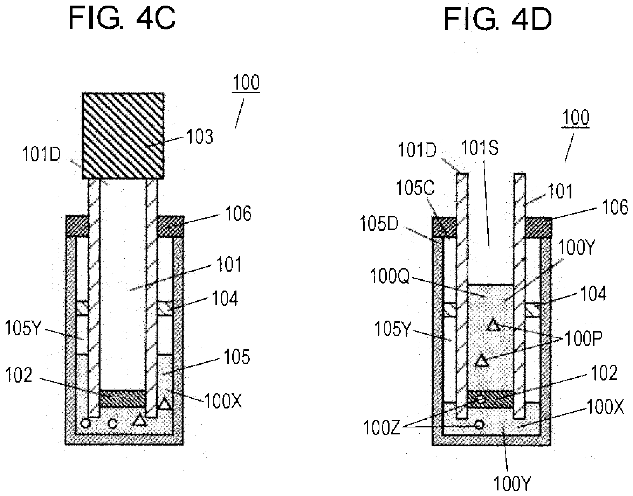

As shown in FIG. 4C, tube 101 is inserted further into container 105. As the insertion of tube 101, hermetically sealed space 105Y in container 105 is gradually decreased while a pressure in hermetically sealed space 105Y is gradually increased. Then, when projection 106 is contacts end portion 105D of opening 105C of container 105, the insertion of tube 101 into container 105 is stopped.

Next, as shown in FIG. 4D, cap 103 mounted on tube 101 is removed from opening end 101D of tube 101. Due to the removal of cap 103, hermetically sealed space 105Y in container 105 is released from the status that the container is compressed by an amount corresponding to a volume of hermetically sealed space 105Y. At this moment, a pressure in hermetically sealed space 105Y intends to return to an atmospheric pressure while a pressure compressed in hermetically sealed space 105Y is maintained so that the pressure tends to push out sample 100X from tube 101. As a result, sample 100X which is pushed out into tube 101 is filtered by passing through filter part 102, and is stored in tube 101 as filtrate 100Q. When sample 100X pushed out into tube 101 passes through filter part 102, solid components 100Z as the filtered-out substances are captured by filter part 102 so that solid components 100Z do not pass through filter part 102 while solid components 100P as the non-filtered-out substances pass through filter part 102 are stored in an upper portion of tube 101 as filtrate 100Q together with liquid component 100Y. According to this embodiment, "a pressure tends to return to an atmospheric pressure while a pressure is maintained" indicates that a rapid change in pressure does not occur, and a change in pressure by an amount corresponding to a volume of the non-filtered-out substances (solid components 100P) accompanying with the returning of the pressure to the atmospheric pressure.

In the case that the diameter of tube 101 is small, sample 100X is sucked into tube 101 through filter part 102 by a capillary phenomenon.

While filtering sample 100X through filter part 102, it is necessary to take into account a surface tension of sample 100X and gravity applied to sample 100X. Sample 100X is stored in container 105 while solid components 100Z as the filtered-out substances are previously contained in sample 100X. When an amount of sample 100X is small, sample 100X may be prepared in container 105 by previously putting a solvent, such as a dilution of the filtered-out substances, in container 105, and then, the filtered-out substances which have been absorbed in, e.g. a sponge are mixed with the solvent.

In the measurement after the separation, the filtered-out substances (solid components 100Z) can be dyed by mixing a dying solution in sample 100X if necessary.

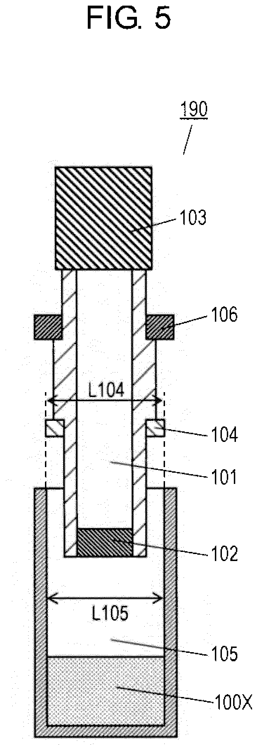

FIG. 5 is a cross-sectional view of another filter device 190 according to Embodiment 1. In FIG. 5, components identical to those of filter device 100 shown in FIG. 1 are denoted by the same reference numerals. As shown in FIG. 5, a diameter of tube 101 may be changed at projection 104 as a boundary. That is, the diameter of a portion of tube 101 directed toward the outside of container 105 from projection 104 is larger than a diameter of a portion of tube 101 directed toward the inside of container 105 from projection 104. Even when projection 104 is inserted into container 105, tube 101 having such a shape does not allow projection 104 to be displaced, and hence, the position of tube 101 and a volume of hermetically sealed space 105Y can be easily controlled. Further, tube 101 having such a shape allows projection 104 to be easily positioned at the time of manufacturing filter device 190. Accordingly, it is preferable to form tube 101 into such a shape.

Effects of filter device 100 according to Embodiment 1 will be described below.

As described above, filter device 100 according to Embodiment 1 includes the capturing-retaining mechanism which is configured to allow sample 100X to pass through filter part 102 at a constant pressure when tube 101 is inserted into container 105. This configuration maintains a pressure in container 105 during the operation of filtering at a constant value without applying an abnormal pressure to filter part 102. That is, in filter device 100, hermetically sealed space 105Y having a pressure not smaller than an atmospheric pressure is formed in container 105. By using a force generated when the pressure returns to the atmospheric pressure (differential pressure), sample 100X is filtered by passing through filter part 102. Accordingly, a force applied to the captured substances which are solid components 100Z captured by filter part 102 does not suddenly increase, and hence, can suppress the occurrence of a phenomenon that the captured substances which are solid components 100Z captured by filter part 102 are removed from filter part 102, thereby stabilizing the capturing performance of filter part 102, and enhancing filter performance of filter device 100.

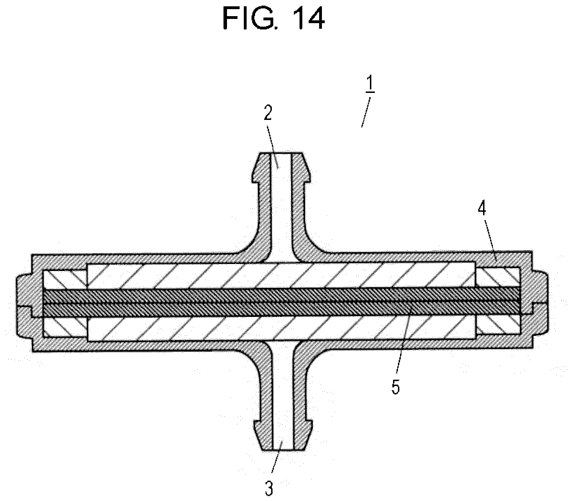

Conventional filter device 1 shown in FIG. 14 cannot prevent an abnormal pressure from being applied to filter part 5, and needs to connect, to filter device 1, a device outside filter device 1 for preventing it. As a result, filter device 1 does not only have a large size but also complicate the mechanism of filter device 1. Accordingly, filter device 1 cannot be used conveniently.

Conventional filter device 1, upon being applied to an extremely small filter device, may cause filter part 5 to lose its capture performance.

For example, in the case that filter device 1 is extremely small, when a sample is continuously pressurized at a constant flow rate, a pressure applied to liquid having blood cells suspended therein is gradually increased. As a result, a pressure applied to captured substances is increased, so that the captured substances captured by filter part 5 may be removed from filter part 5 to outlet 3.

Alternatively, for example, all of liquid having blood cells suspended therein passes through filter part 5, and hence, the substances captured by filter part 5 are removed from filter part 5 due to release or removal of the captured substances, and the captured substances are removed to outlet 3. As a result, filter performance of filter device 1 is deteriorated.

Filter device 100 according to Embodiment 1 includes the capturing-retaining mechanism which can control a pressure applied to sample 100X. Even when filter device 100 is not connected to a device for controlling a pressure provided outside filter device 100, it is possible to control the pressure only by filter device 100. Further, filter device 100 does not require the complicated mechanism, and hence, the operation of filter device 100 is performed simply and easily.

As described above, filter device 100 is configured to filter sample 100X containing liquid component 100Y and solid components 100Z mixed with liquid component 100Y. Filter device 100 includes container 105 configured to store sample 100 therein, tube 101 configured to be inserted into container 105, filter part 102 provided inside tube 101, and a capturing-retaining mechanism. When tube 101 is inserted into container 105, filter part 102 is configured to allow the stored sample 100 to pass through filter part 102 so as to capture solid components 100Z and to allow liquid component 100Y to pass through filter part 102. The capturing-retaining mechanism (projection 104) is configured to prevent the captured solid components 100Z from being removed from filter part 102 when sample 100X passes through filter part 102.

The capturing-retaining mechanism (projection 104) may be configured such that an average mobility of solid components 100Z in filter part 102 when sample 100X passes through filter part 102 is constant.

The capturing-retaining mechanism (projection 104) may be configured such that a pressure applied to sample 100X when sample 100X passes through filter part 102 is constant.

Projection 104, the capturing-retaining mechanism, being provided on an outer circumference of tube 101 and surrounding the outer circumference of tube 101. Projection 104 has an outermost diameter not smaller than an inner diameter of container 105.

Container 105 has an opening having tube 101 inserted therein. The capturing-retaining mechanism further includes projection 106 provided on tube 101. Projection 106 being configured to contact an end portion of container 105 facing the opening so as to prevent tube 101 from being inserted into container 105 by a depth not smaller than a predetermined depth.

### EXEMPLARY EMBODIMENT 2

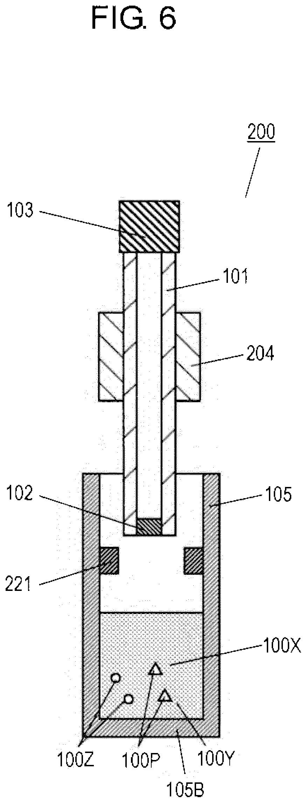

FIG. 6 is a cross-sectional view of filter device 200 which is a blood filter device according to Exemplary Embodiment 2. In FIG. 6, components identical to those of filter device 100 according to Embodiment 1 shown in FIGs. 1 to 5 are denoted by the same reference numerals. Filter device 200 includes projection 204 and stopper 221 instead of projections 104 and 106 of filter device 100 according to Embodiment 1. Filter device 200 includes a capturing-retaining mechanism configured to prevent solid components 100Z which are captured substances captured by filter part 102 from being removed from filter part 102 due to release or removal of solid components 100Z when tube 101 is inserted into container 105. As shown in FIG. 6, for example, the capturing-retaining mechanism is configured such that a portion of sample 100X does not pass through filter part 102 and remains in container 105 when tube 101 is inserted into container 105.

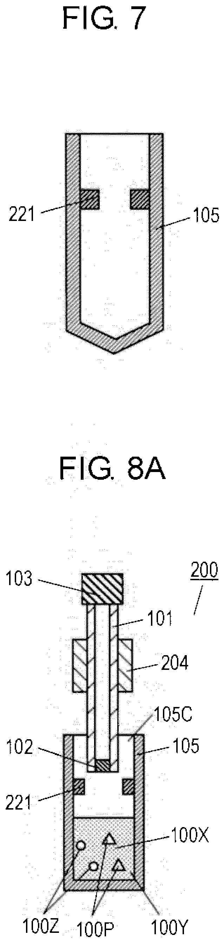

As a volume of container 105 is larger, a pressure in container 105 can be more easily adjusted during filtering. In this case, however, the amount of the portion of sample 100X which remains in container 105 is increased. FIG. 7 is a cross-sectional view of another container 105 of filter device 200 according to Embodiment 2. A bottom surface of container 105 has a conical shape such that the center of the bottom surface projects toward the outside. This shape can reduce the amount of the remaining portion of sample 100X. The bottom surface of container 105 may have a hemispherical shape instead.

In the case that the bottom surface of container 105 has a conical shape, when a tapered angle (an angle formed by a side surface of a conical shape and the bottom surface) is too large, that is, when a vertex angle θ of the conical shape is small, the amount of the remaining portion of sample 100X becomes large. Accordingly, such a shape is not preferable.

The capturing-retaining mechanism of filter device 200 according to Embodiment 2 is configured such that a portion of sample 100X does not pass through filter part 102 and remains in container 105 when tube 101 is inserted into container 105. As shown in FIG. 6, the capturing-retaining mechanism of filter device 200 includes projection 204 formed on an outer circumference of tube 101 and stopper 221 extending from an inner wall of container 105.

When tube 101 is inserted into container 105, projection 204 is stopped with stopper 221 so that tube 101 is not inserted further into container 105.

In filter device 200 according to Embodiment 2, projection 204 is stopped at a plane on stopper 221. However, undulations may be formed in projection 204 and stopper 221, and these undulations may be fitted to each other. Further, projection 204 and stopper 221 may have a threaded engagement structure.

The position of projection 204 is determined such that sample 100X put in container 105 does not go beyond stopper 221 when tube 101 is inserted into container 105.

When filter part 102 is inserted into container 105 and is stopped at the end portion of container 105, a lower surface of filter part 102 does not preferably reach bottom 105B of container 105. When the lower surface of filter part 102 contacts bottom 105B of container 105, filtering efficiency is deteriorated. Accordingly, a distance from a bottom of container 105 to stopper 221 is larger than a length from projection 204 to a distal end of filter part 102.

A size of projection 204 is determined to be a size which allows projection 204 to be inserted into container 105 to prevent that the occurrence of leakage of sample 100X or air from an outer periphery of projection 204 when tube 101 is inserted into container 105.

An operation of filter device 200 will be described below. FIGs. 8A to 8C are cross-sectional views of filter device 200 for illustrating a filtering method used with filter device 200.

As shown in FIG. 8A, sample 100X is put in container 105. Then, tube 101 is inserted into container 105 through upper opening 105C of container 105 provided at the upper part of container 105.

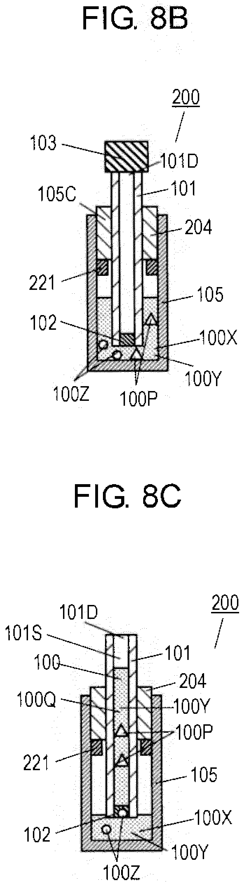

As shown in FIG. 8B, tube 101 is inserted into container 105 until projection 204 fixed to tube 101 is stopped with stopper 221 provided inside container 105.

As shown in FIG. 8C, when opening end 101D is released by removing cap 103 from tube 101, sample 100X is filtered by passing through filter part 102. Filtrate 100Q which is sample 100X filtered through filter part 102 is stored in inner space 101S of tube 101 at an upper portion of tube 101. Filtrate 100Q contains non-filtered-out substances (solid components 100P) and liquid component 100Y, and does not contain the filtered-out substances (solid components 100Z).

In the case that the diameter of tube 101 is small, sample 100X is sucked into tube 101 through filter part 102 by a capillary phenomenon.

A filtering speed and an amount of sample 100X passing through filter part 102 both of which are dependent on a pressure (pressure-dependent) can be controlled by controlling a compression amount of air in the container. At this moment, the own weight of sample 100X is neglected, and hence, a pressure inside container 105 is maintained approximately constant.

The filtering is finished while a portion of sample 100X remains in container 105.

A volume compressed when tube 101 is inserted into container 105 is preferably not larger than a volume of sample 100X which passes through filter part 102.

In filtering sample 100X, it is necessary to take into account a surface tension of sample 100X and the gravity of sample 100X. The surface tension of sample 100X generated between sample 100X and an inner wall of tube 101 prevents a portion of sample 100X from passing through filter part 102 and being filtered. The compression volume is not larger than the sum of a volume of sample 100X which is filtered through filter part 102 and a volume of the portion of sample 100X which is not filtered.

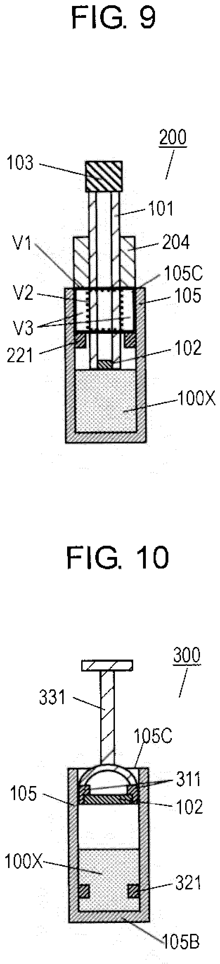

FIG. 9 is a cross-sectional view of filter device 200. As shown in FIG. 9, the compression volume is volume V3 which is a difference obtained by subtracting volume V2 from volume V1. Volume V2 ranges from opening 105C of container 105 to a portion of tube 101 positioned inside container 105. Volume V1 ranges from opening 105C of container 105 to stopper 221.

An amount of sample 100X which can pass through filter part 102 is smaller than a total amount of sample 100X put into container 105. That is, the sum of a volume of sample 100X which is filtered through filter part 102 and a volume of the portion of sample 100X which is not filtered is the volume of sample 100X.

Due to the above-mentioned configuration, in filter device 200, the filtering is finished in a state where the portion of sample 100X remains in container 105.

Sample 100X which is not filtered and thus remaining in container 105 has a volume in which a height of an upper surface of sample 100X remaining in container 105 is not lower than a height from a bottom of container 105 to a lower surface of filter part 102 after tube 101 is inserted into container 105. That is, sample 100X contacts filter part 102 even after the filtering is finished. As a result, sample 100X always contacts filter part 102.

Sample 100X is stored in container 105 in a state where solid components 100Z as the filtered-out substances are previously contained in sample 100X. In the case that the an amount of sample 100X is small, sample 100X may be prepared inside container 105 by previously putting a solvent, such as a dilution of the filtered-out substances, in container 105 and then by mixing, with the solvent, the filtered-out substances which have been absorbed in a sponge.

An effect of filter device 200 according to Embodiment 2 will be described below.

As described above, in filter device 200 according to Embodiment 2, air filling a space from opening 105C of container 105 to stopper 221 can be compressed by stopper 221 provided inside container 105 and projection 204 formed on tube 101 which is inserted into container 105. Accordingly, sample 100X can be filtered by causing sample 100X to pass through filter part 102 by a pressure difference between the compressed air and an atmospheric air.

A length from filter part 102 to projection 204 is shorter than a length from the bottom of container 105 to stopper 221. This configuration can allow sample 100X to remain in container 105 even after the filtering is finished. Accordingly, sample 100X can be filtered without losing capture performance of filter part 102.

In filter device 1 shown in FIG. 14, when all the sample is filtered, a capture performance of filter part 5 may be lost so that the captured substances (solid components 100Z) which are captured by filter part 5 may be removed from filter part 5 due to release or removal of the captured substances, and are removed to an outlet. This is because, when a phase of the pores in filter part 5 changes from a liquid phase to a gas phase, a viscosity of the sample is lowered, and hence, a flow speed (flow amount) of the sample is increased, thereby a force applied to the captured substances is increased and exceeds a force to absorb the captured substances into filter part 5. Alternatively, in the case that the captured substances are specimens, particularly specimens derived from a living body, a deformation amount of specimens per se is accelerated, and hence, the capture performance is deteriorated. Further, hydrophilicity of a capturing surface of filter part 5 is lowered so that a surface tension is greatly decreased, thereby a state of the capturing surface of filter part 5 is remarkably changed. Such a change is considered to bring about a change in adhesiveness (charge coupling, chemical bonding or the like) of the surface.

In filter device 200 according to Embodiment 2, pores formed in filter part 102 are always filled with liquid component 100Y during filtering. This configuration allows a portion of sample 100X to remain in container 105 without filtering all sample 100X through filter part 102, so that sample 100X can remain in filter part 102.

Since the inside of filter part 102 is filled with sample 100X, a force applied to the captured substances (solid components 100Z) captured by filter part 102 is not suddenly increased. Accordingly, the filtering can be finished while preventing the captured substances from being removed from filter part 102. As a result, a filter performance of filter device 200 can be enhanced.

In filter device 200 according to Embodiment 2, a pressure or a flow rate at which filtering is performed can be controlled by controlling a compression amount of air in a space from opening 105C of container 105 to stopper 221 and a speed at which air is leaked from cap 103. Accordingly, the stability of capture performance of filter part 102 is enhanced.

Cap 103 which is easily removable is mounted on opening end 101D of tube 101 opposite to opening end 101C to which filter part 102 is joined.

Cap 103 can increase an inner pressure of container 105 without causing filter part 102 to contact sample 100X when tube 101 is inserted into container 105, hence reducing a length of container 105. Further, the inner pressure of container 105 rises to a predetermined pressure, and then, cap 103 is removed, hence allowing sample 100X to be filtered at a predetermined pressure. Accordingly, filter part 102 can easily exert capture performance stably.

For the reason similar to the above, filter device 200 may include a metal seal, a rubber plug, a leak valve, or a leak hole which can close and open opening end 101D of tube 101 instead of cap 103.

In filter device 200 according to Embodiment 2, the capturing-retaining mechanism which is configured such that a portion of sample 100X does not pass through filter part 102 and remains in container 105 includes projection 204 formed on the outer circumference of tube 101 and stopper 221 which extends from the inner wall of container 105 and stops projection 204. Filter device 200 according to Embodiment 2 is not limited to the configuration which includes a control mechanism for causing sample 100X to remain inside container 105 as described above. That is, filter device 200 may include a control mechanism which causes sample 100X to take out from container 105.

As described above, the capturing-retaining mechanism is configured such that a portion of sample 100X does not pass through filter part 102 and remains in container 105 when tube 101 is inserted into container 105.

The capturing-retaining mechanism includes projection 204 formed on the outer circumference of tube 101 and stopper 221 provided on the inner wall of container 105. Stopper 221 is configured to contact projection 204 when tube 101 is inserted into container 105.

A compression volume when tube 101 is inserted into container 105 is not larger than an amount of sample 100X which passes through filter part 102.

### EXAMPLE 1

FIG. 10 is a cross-sectional view of filter device 300 according to Example 1 which does not form part of the invention but is useful for understanding the invention. In FIG. 10, components identical to those of filter devices 100 and 200 according to the first Embodiments 1 and 2 shown in FIGs. 1 to 9 are denoted by the same reference numerals.

Filter device 300 according to Example 1 includes insertion mechanism 331 for inserting filter part 102 into container 105 instead of tube 101 of filter device 100 according to Embodiment 1 or tube 101 of filter device 200 according to Embodiment 2. Filter part 102 is inserted into container 105 by inserting insertion mechanism 331 into container 105 so that filter part 102 can be inserted into sample 100X stored in container 105. Sample 100X is filtered by passing through filter part 102 to acquire filtrate 100Q. Filtrate 100Q is collected in an upper portion of container 105, that is, on a side of filter part 102 opposite to a bottom of container 105.

An outer periphery of filter part 102 is covered by wall part 311. Filter part 102 is joined to wall part 311 so as to prevent the occurrence of leakage between filter part 102 and wall part 311. Thin plate 108 having apertures 107 therein shown in FIGs. 2A and 2B may be provided on wall part 311. In this case, filter part 102 is provided on a lower surface or an upper surface of the thin plate. The thin plate provided on the lower surface or the upper surface of filter part 102 increases the strength of filter part 102. Wall part 311 and the thin plate may preferably have hydrophilicity.

Insertion mechanism 331 can pressurize filter part 102 from above wall part 311. Insertion mechanism 331 is joined to filter part 102. By pushing insertion mechanism 331, filter part 102 is inserted into container 105.

For example, a plunger may be used as insertion mechanism 331. A pushing rod is provided at wall part 311 of filter part 102. By pushing the pushing rod, filter part 102 can be inserted into container 105.

Filter device 300 includes a capturing-retaining mechanism configured such that, when insertion mechanism 331 is inserted into container 105 for storing sample 100X, a portion of sample 100X put in container 105 does not pass through filter part 102 and remains in container 105.

The capturing-retaining mechanism allowing sample 100X to remain in container 105 includes, e.g. stopper 321 provided on an inner wall of container 105. Stopper 321 is configured to stop filter part 102 so as to prevent filter part 102 from contacting the bottom surface of container 105. Stopper 321 is configured to stop wall part 311 such that wall part 311 contacts stopper 321 when filter part 102 is inserted into container 105. Stopper 321 is provided at a position not lower than a height from the bottom surface of container 105 at which a predetermined volume of the remaining portion of the sample can be obtained.

Stopper 321 provided at the position away from bottom 105B of container 105 where a predetermined amount of the remaining liquid can be obtained stop the insertion of filter part 102 into container 105. Accordingly, filter 300 can perform filtering of sample 100X without losing capture performance of filter part 102.

In filter device 300 according to Example 1, the non-filtered-out substances (solid components 100P) pass through filter part 102 and are stored in an upper portion of container 105. Accordingly, after filter part 102 is inserted into container 105, the non-filtered-out substances above filter part 102 and the non-filtered-out substances below filter part 102 inside container 105 leak only through filter part 102, and the non-filtered-out substances do not leak through wall part 311.

As described above, insertion mechanism 331 is configured to insert filter part 102 into container 105 to cause the stored sample 100X pass through filter part 102 such that solid components 100Z are captured by filter part 102 and liquid component 100Y passes through filter part 102.

Stopper 321, the capturing-retaining mechanism, extends from the inner wall of container 105 so as to stop filter part 102.

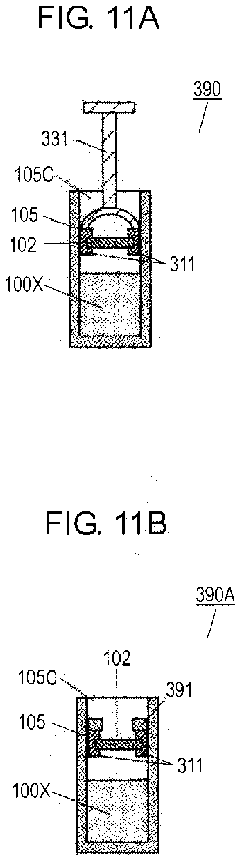

FIG. 11A is a cross-sectional view of another filter device 390 according to Example 1. In FIG. 11A, components identical to those of filter device 300 shown in FIG. 10 are denoted by the same reference numerals. In filter device 390 shown in FIG. 11A, the capturing-retaining mechanism which allows sample 100X to remain in container 105 includes wall part 311 configured to prevent filter part 102 from reaching a bottom of container 105. Wall part 311 projects toward the bottom of container 105 from filter part 102. That is, a height of a lower surface of filter part 102 (a lower surface of the thin plate in the case where filter part 102 includes the thin plate) is higher than a height of a lower surface of wall part 311. That is, filter device 390 has a structure in which the lower surface of wall part 311 firstly contacts the bottom of container 105 by inserting filter part 102 into container 105. This configuration allows sample 100X to remain in a well portion formed by wall part 311.

FIG. 11B is a cross-sectional view of still another filter device 390A according to Example 1. In FIG. 11B, components identical to those of filter device 390 shown in FIG. 11A are denoted by the same reference numerals. Filter device 390A shown in FIG. 11B includes weight 391 mounted on wall part 311 instead of insertion mechanism 331 of filter device 390 shown in FIG. 11A. Weight 391 mounted on wall part 311 can push filter part 102 by gravity. As a result, filter part 102 can be inserted into container 105 at a constant speed. Thus, weight 391 functions as an insertion mechanism for inserting filter part 102 into container 105.

Weight 391 may preferably have a weight not smaller than the sum of a buoyancy of sample 100X and a fluid resistance. This configuration allows filter part 102 to sink in sample 100X to the bottom of container 105 while pressurizing sample 100X. Accordingly, sample 100X can be filtered by merely inserting filter part 102 having weight 391 and wall part 311 into container 105.

As described above, the insertion mechanism configured to insert filter part 102 into container 105 includes weight 391 mounted on filter part 102.

### EXAMPLE 2

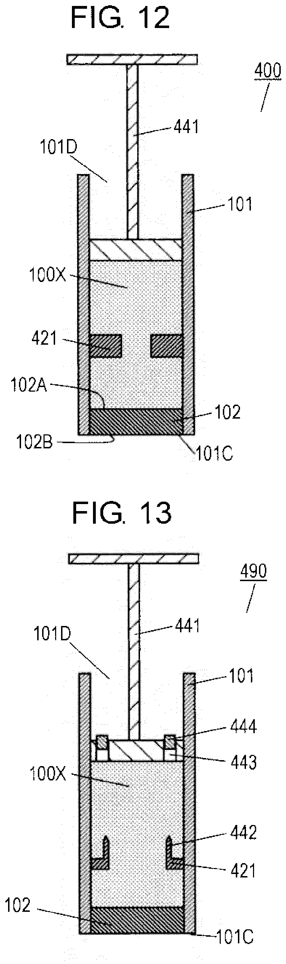

FIG. 12 is a cross-sectional view of filter device 400 according to Example 2 which does not form part of the invention but is useful for understanding the invention. In FIG. 12, components identical to those of the filter devices according to Embodiments 1 and 2 and Example 1 shown in FIGs. 1 to 11B are denoted by the same reference numerals. Filter device 400 according to Example 2 does not include container 105 configured to store sample 100X of the filter devices according to Embodiments 1 and 2 and Example 1. Instead, filter device 400 is configured such that tube 101 stores sample 100X. Filter part 102 is provided inside tube 101 so as to close tube 101. Sample 100X is stored above filter part 102 in tube 101, that is, in a space formed in the direction from filter part 102 toward opening end 101D of tube 101. By pressurizing the inside of tube 101 by pressurizing mechanism 441 from above tube 101, that is, from opening end 101D of tube 101, sample 100X is filtered by causing sample 100X to pass through filter part 102. Filter device 400 includes a capturing-retaining mechanism configured to prevent a portion of sample 100X stored between filter part 102 and pressurizing
mechanism 441 from passing through filter part 102 and to cause the portion to remain between filter part 102 and pressurizing mechanism 441 when pressurizing mechanism 441 is inserted into tube 101.

As pressurizing mechanism 441 for pressurizing the inside of tube 101 from above tube 101, a plunger provided with a pushing rod shown in FIG. 12 can be used, for example.

To effectively use the inside of tube 101, filter part 102 may preferably be provided at opening end 101C of tube 101.

The capturing-retaining mechanism which allows sample 100X to remain in tube 101 is, for example, stopper 421 extending from an inner wall of tube 101. Stopper 421 is configured to stop pressurizing mechanism 441. A distance from filter part 102 to stopper 421 is determined according to a predetermined amount of the portion of sample 100X remaining in tube 101.

A tensile force generated by a surface tension is applied to sample 100X which is located between filter part 102 and pressurizing mechanism 441 stopped with stopper 421 on an inner wall of tube 101 and on a surface of pressurizing mechanism 441. As a result, sample 100X located between filter part 102 and pressurizing mechanism 441 stopped with stopper 421 remains in tube 101.

The amount of the portion of sample 100X remaining in tube 101 between filter part 102 and pressurizing mechanism 441 stopped with stopper 421 depends on an inner diameter of tube 101. Accordingly, a surface tension of remaining sample 100X may preferably be larger than the gravity of sample 100X. In this case, the inner wall of tube 101 and the surface of pressurizing mechanism 441 may preferably have hydrophilicity.

In filter device 400 shown in FIG. 12, an air phase may not preferably formed in an interface between sample 100X and pressurizing mechanism 441. That is, pressurizing mechanism 441 may preferably contact sample 100X directly.

In order to prevent an air phase from being formed in the interface between sample 100X and pressurizing mechanism 441, a hydrophobic membrane may be preferably provided on a surface of pressurizing mechanism 441 contacting sample 100X (a lower surface of pressurizing mechanism 441 shown in FIG. 12). Although sample 100X in a liquid phase cannot pass through the hydrophobic membrane, an air phase can pass through the hydrophobic membrane. As a result, pressurizing mechanism 441 can directly contact sample 100X at the interface between sample 100X and pressurizing mechanism 441.

In order to prevent an air phase from being formed in the interface between sample 100X and pressurizing mechanism 441, a speed at which pressurizing mechanism 441 is inserted may preferably be identical to a speed of leakage from tube 101 and pressurizing mechanism 441.

This configuration allows the interface of pressurizing mechanism 441 to contact sample 100X directly.

Alternatively, sample 100X may be sucked into tube 101 from the outside of tube 101 through filter part 102, and sample 100X is pushed out after a distal end of filter part 102 is cleaned.

The filtering condition shown in FIG. 12 can be obtained also by putting sample 100X into tube 101 up to opening end 101D.

Sample 100X directly contacts pressurizing mechanism 441. A force in the upward direction generated by a surface tension on the inner wall of tube 101 is not smaller than the product obtained by multiplying a mass of sample 100X by the gravitational acceleration. This configuration allows sample 100X to remain in tube 101.

Filter part 102 has surfaces 102B and 102A. Surface 102B faces opening end 101C of tube 101. Surface 102A faces opening end 101D. As shown in FIG. 2A, a thin plate having apertures provided therein may be provided on surface 102A or surface 102B of filter part 102. In this case, a surface tension acts on an outer periphery of a distal end of the aperture (outlet for filtrate 100Q). A tensile force generated by a surface tension may become larger than the gravity applied to a droplet of sample 100X. In this case, whether or not the droplet is dropped, that is, whether or not sample 100X leaks through the aperture is relevant to a diameter of a neck portion of the droplet formed at the distal end of the aperture. A surface tension is a force which acts on the circumference, and hence, the droplet is not dropped if a tensile force generated by a surface tension of the neck portion is larger than the gravity applied to the droplet.

As described above, in filter device 400 according to Example 2, in a state where pressurizing mechanism 441 stopped with stopper 421 contacts sample 100X completely, a surface tension of sample 100X acts on the inner wall of tube 101 or the surface of pressurizing mechanism 441 contacting sample 100X, and hence, allows sample 100X to remain in tube 101. As a result, filter device 400 can filter sample 100X without losing capture performance of filter part 102.

The remaining amount of sample 100X can be controlled by adjusting a distance from filter part 102 to stopper 421, and hence, it is possible to allow an extremely small amount of sample 100X to remain in tube 101.

In filter device 400 according to Example 2, sample 100X can remain in tube 101 even when pressurizing mechanism 441 does not contact sample 100X completely, that is, even when an air phase is formed between pressurizing mechanism 441 and sample 100X.

As described above, tube 101 is configured to store sample 100X therein. Filter part 102 is configured to close tube 101. Pressurizing mechanism 441 is configured to pressurize the stored sample 100X so as to cause the stored sample 100X to pass through the filter part 102 such that the solid components 100Z are captured by the filter part 102 and the liquid component 100Y passes through the filter part 102. The capturing-retaining mechanism is configured to prevent a portion of the stored sample 100X from passing through the filter part 102 and to cause the portion of the stored sample 100X remain between the filter part 102 and the pressurizing mechanism, such that the captured solid components 100Z are not removed from the filter part when the sample 100X passes through the filter part 102.

The capturing-retaining mechanism, stopper 441, extends from an inner wall of tube 101 as to stop the pressurizing mechanism 441.

FIG. 13 is a cross-sectional view of another filter device 490 according to Example 2 which does not form part of the invention but is useful for understanding the invention. In FIG. 13, components identical to those of filter device 400 shown in FIG. 12 are denoted by the same reference numerals. Filter device 490 shown in FIG. 13 further includes projections 442 which extend toward opening end 101D in the direction in which tube 101 extends from stopper 421 of filter device 400 shown in FIG. 12. Apertures 443 are formed in pressurizing mechanism 441. Filter device 490 further includes movable plugs 444 which close apertures 443.

Movable plug 444 is made of a movable rubber plug, cork plug, metal plug, resin plug or a lid, or a breakable membrane, such as a resin membrane, a rubber membrane, a metal membrane, or a fiber membrane.

Pressurizing mechanism 441 compresses an air between sample 100X and pressurizing mechanism 441.

The above-mentioned the air which is compressed due to the insertion of pressurizing mechanism 441 into tube 101 is released to an atmospheric air by removing movable plugs 444 with projections 442. As a result, the difference in pressure between inside and outside of tube 101 is eliminated, and hence, allows sample 100X to remain in tube 101.

Projections 442 may preferably extend toward opening end 101D in the direction in which a side wall of tube 101 extends. That is, apertures 443 may not preferably contact projections 442. In order to remove movable plugs 444 from aperture 443 of pressurizing mechanism 441, a length of projections 442 is larger than a length of apertures 443.

To allow the compressed air between sample 100X and pressurizing mechanism 441 to leak through apertures 443, an inner diameter of aperture 443 is larger than a diameter of projection 442.

As described above, filter device 490 according to Example 2 includes movable plugs 444 which are provided on pressurizing mechanism 441 and contact an air inside tube 101. While pressurizing sample 100X with pressurizing mechanism 441, projections 442 formed on stopper 421 are inserted into apertures 443 provided in pressurizing mechanism 441. When movable plugs 444 are removed by projections 442, the air compressed inside tube 101 can be released to an atmospheric air, and hence, allows sample 100X to remain in tube 101 due to a pressure equilibrium.

### INDUSTRIAL APPLICABILITY

A filter device according to the present invention is applicable to a device for extracting only a particular substance from a sample.

### REFERENCE MARKS IN DRAWINGS

- 100X: sample
- 100Y: liquid component
- 100Z: solid component
- 101: tube
- 102: filter part
- 103: cap
- 104: projection (first projection)
- 105: container
- 106: projection (second projection)
- 107: aperture
- 221: stopper
- 331: insertion mechanism
- 441: pressurizing mechanism
- 442: projection
- 443: aperture
- 444: movable plug

## Claims

1. A blood filter device configured to filter a sample (100X) containing a liquid component (100Y) and a plurality of solid components (100Z) mixed with the liquid component (100Y), the blood filter device (100, 200) comprising:
a container (105) configured to store the sample (100X) therein;
a tube (101) configured to be inserted into the container (105);
a filter part (102) provided inside the tube (101), wherein, when the tube (101) is inserted into the container (105), the filter part (102) is configured to allow the stored sample (100X) to pass through the filter part (102) so as to capture the plurality of solid components (100Z) and to allow the liquid component (100Y) to pass through the filter part (102);
a capturing-retaining mechanism (104, 106, 204, 221) configured to prevent the plurality of captured solid components (100Z) from being removed from the filter part (102) when the sample (100X) passes through the filter part (102); and
a cap (103) provided at an opening end (101D) of the tube (101) opposite to an opening end (101C) to which the filter part (102) is joined and closing the opening end (101D) of the tube (101), the cap (103) being configured to be removable from the opening end (101D) of the tube (101), wherein the cap (103) is configured to hermetically seal the container (105) to increase an inner pressure of the container (105) when the tube (101) is inserted into the container (105);
wherein the container (105) has an opening (105C) having the tube (101) inserted therein, and
wherein the capturing-retaining mechanism (104, 106, 204, 221) is structured such that:
the capturing-retaining mechanism (104, 106, 204, 221) includes
a first projection (104) provided on an outer circumference of the tube (101) and surrounding the outer circumference of the tube (101), the first projection (104) having an outermost diameter not smaller than an inner diameter of the container (105). and
a second projection (106) provided on the tube (101), the second projection (106) being configured to contact an end portion of the container (105) facing the opening (105C) so as to prevent the tube (101) from being inserted into the container (105) by a depth not smaller than a predetermined depth or
the capturing-retaining mechanism (104, 106, 204, 221) includes:
a projection (204) provided on an outer circumference of the tube (101); and
a stopper (221) provided on an inner wall of the container (105), the stopper (221) being configured to contact the projection (204) when the tube (101) is inserted into the container (105).

2. The blood filter device according to claim 1, wherein the capturing-retaining mechanism (104, 106, 204, 221) is configured such that a portion of the sample (100X) does not pass through the filter part (102) and remains in the container (105) when the tube (101) is inserted into the container (105).

3. The blood filter device according to claim 2, wherein a compressed volume of the container (105) when the tube (101) is inserted into the container (105) is not larger than an amount of the sample (100X) that passes through the filter part (102).

## Patentansprüche

1. Blutfilter-Vorrichtung, die so eingerichtet ist, dass sie eine Probe (100X) filtert, die eine flüssige Komponente (100Y) und eine Vielzahl mit der flüssigen Komponente (100Y) vermischter fester Komponenten (100Z) enthält, wobei die Blutfilter-Vorrichtung (100, 200) umfasst:
einen Behälter (105), der zum Aufbewahren der Probe (100X) darin eingerichtet ist;
eine Röhre (101), die zum Einführen in den Behälter (105) eingerichtet ist;
einen Filter-Teil (102), der sich im Inneren der Röhre (101) befindet, wobei der Filter-Teil (102) so eingerichtet ist, dass er, wenn die Röhre (101) in den Behälter (105) eingeführt ist, zulässt, dass die aufbewahrte Probe (100X) durch den Filter-Teil (102) hindurchtritt, um so die Vielzahl fester Komponenten (100Z) einzufangen, und zulässt, dass die flüssige Komponente (100Y) durch den Filter-Teil (102) hindurchtritt;
einen Mechanismus (104, 106, 204, 221) zum Einfangen und Halten, der so eingerichtet ist, dass er verhindert, dass die Vielzahl eingefangener fester Komponenten (100Z) aus dem Filter-Teil (102) entfernt werden, wenn die Probe (100X) durch den Filter-Teil (102) hindurchtritt; sowie
eine Kappe (103), die sich an einem Öffnungs-Ende (101D) der Röhre (101) befindet, das einem Öffnungs-Ende (101C) gegenüberliegt, mit dem der Filter-Teil (102) verbunden ist, und die das Öffnungs-Ende (101D) der Röhre (101) verschließt, wobei die Kappe (103) so eingerichtet ist, dass sie von dem Öffnungs-Ende (101D) der Röhre (101) entfernt werden kann, und die Kappe (103) so eingerichtet ist, dass sie den Behälter (105) hermetisch abdichtet, um einen Innendruck des Behälters (105) zur erhöhen, wenn die Röhre (101) in den Behälter (105) eingeführt wird;
wobei der Behälter (105) eine Öffnung (105C) aufweist, in die die Röhre (101) eingeführt ist, und
der Mechanismus (104, 106, 204, 221) zum Einfangen und Halten so aufgebaut ist, dass der Mechanismus (104, 106, 204, 221) zum Einfangen und Halten enthält:
einen ersten Vorsprung (104), der sich an einem Außenumfang der Röhre (101) befindet und den Außenumfang der Röhre (101) umschließt, wobei der erste Vorsprung (104) einen größten Außendurchmesser hat, der nicht kleiner ist als ein Innendurchmesser des Behälters (105), sowie
einen zweiten Vorsprung (106), der sich an der Röhre (101) befindet, wobei der zweite Vorsprung (106) so eingerichtet ist, dass er mit einem Endabschnitt des Behälters (105) in Kontakt kommt, der der Öffnung (105C) zugewandt ist, um zu verhindern, dass die Röhre (101) um eine Tiefe in den Behälter (105) eingeführt wird, die nicht kleiner ist als eine vorgegebene Tiefe, oder
der Mechanismus (104, 106, 204, 221) zum Einfangen und Halten enthält:
einen Vorsprung (204), der sich an einem Außenumfang der Röhre (101) befindet; und
einen Anschlag (221), der sich an einer Innenwand des Behälters (105) befindet, wobei der Anschlag (221) so eingerichtet ist, dass er mit dem Vorsprung (204) in Kontakt kommt, wenn die Röhre (101) in den Behälter (105) eingeführt wird.

2. Blutfilter-Vorrichtung nach Anspruch 1, wobei der Mechanismus (104, 106, 204, 221) zum Einfangen und Halten so eingerichtet ist, dass ein Teil der Probe (100X) nicht durch den Filter-Teil (102) hindurchtritt und in dem Behälter (105) verbleibt, wenn die Röhre (101) in den Behälter (105) eingeführt wird.

3. Blutfilter-Vorrichtung nach Anspruch 2, wobei ein verdichtetes Volumen des Behälters (105), wenn die Röhre (101) in den Behälter (105) eingeführt wird, nicht größer ist als eine Menge der Probe (100X), die durch den Filter-Teil (102) hindurchtritt.

## Revendications

1. Dispositif de filtre sanguin configuré pour filtrer un échantillon (100X) contenant un composant liquide (100Y) et une pluralité de composants solides (100Z) mélangés avec le composant liquide (100Y), le dispositif de filtre sanguin (100, 200) comprenant:
un récipient (105) configuré pour stocker l'échantillon (100X) dans celui-ci;
un tube (101) configuré pour être introduit dans le conteneur (105);
une partie filtre (102) prévue à l'intérieur du tube (101), dans laquelle, lorsque le tube (101) est introduit dans le récipient (105), la partie filtre (102) est configurée pour laisser passer l'échantillon stocké (100X) à travers la partie filtre (102) de manière à capturer la pluralité de composants solides (100Z) et à permettre au composant liquide (100Y) de traverser la partie filtre (102);
un mécanisme de capture-retenue (104, 106, 204, 221) configuré pour empêcher la pluralité des composants solides capturés (100Z) d'être retirés de la partie filtre (102) lorsque l'échantillon (100X) passe à travers la partie filtre (102); et
un capuchon (103) prévu à une extrémité d'ouverture (101D) du tube (101) opposée à une extrémité d'ouverture (101C) à laquelle la partie filtre (102) est jointe et fermant l'extrémité d'ouverture (101D) du tube (101), le capuchon (103) étant configuré pour être amovible de l'extrémité d'ouverture (101D) du tube (101), dans lequel le capuchon (103) est configuré pour sceller hermétiquement le récipient (105) afin d'augmenter une pression interne du récipient (105) lorsque le tube (101) est introduit dans le récipient (105);
dans lequel le récipient (105) comporte une ouverture (105C) dans laquelle est introduit le tube (101), et
dans lequel le mécanisme de capture-retenue (104, 106, 204, 221) est structuré de telle sorte que:
le mécanisme de capture-retenue (104, 106, 204, 221) comprend
une première protubérance (104) prévue sur une circonférence extérieure du tube (101) et entourant la circonférence extérieure du tube (101), la première protubérance (104) ayant un diamètre le plus externe supérieur ou égal à un diamètre intérieur du récipient (105), et
une seconde protubérance (106) prévue sur le tube (101), la seconde protubérance (106) étant configurée pour entrer en contact avec une partie d'extrémité du récipient (105) faisant face à l'ouverture (105C) de manière à empêcher l'introduction du tube (101) dans le récipient (105) d'une profondeur supérieure ou égale à une profondeur prédéterminée ou bien le mécanisme de capture-retenue (104, 106, 204, 221) comprend:
une protubérance (204) prévue sur une circonférence extérieure du tube (101); et
un bouchon (221) prévu sur une paroi interne du récipient (105), le bouchon (221) étant configuré pour venir au contact de la protubérance (204) lorsque le tube (101) est introduit dans le récipient (105).

2. Dispositif de filtre sanguin selon la revendication 1, dans lequel le mécanisme de capture-retenue (104, 106, 204, 221) est configuré de sorte qu'une partie de l'échantillon (100X) ne traverse pas la partie filtre (102) et reste dans le récipient (105) lorsque le tube (101) est introduit dans le récipient (105).

3. Dispositif de filtration de sang selon la revendication 2, dans lequel un volume comprimé du récipient (105) lorsque le tube (101) est introduit dans le récipient (105) est inférieur ou égal à une quantité de l'échantillon (100X) qui traverse la partie filtre (102).
